(19) Europäisches Patentamt
European Patent Office
Office européen des brevets

(11) **EP 2 040 775 B1**

(12) **EUROPÄISCHE PATENTSCHRIFT**

(45) Veröffentlichungstag und Bekanntmachung des Hinweises auf die Patenterteilung:
**03.07.2013 Patentblatt 2013/27**

(21) Anmeldenummer: 07765161.0

(22) Anmeldetag: **11.07.2007**

(51) Int Cl.:
*A61M 1/16* *(2006.01)*

(86) Internationale Anmeldenummer:
**PCT/EP2007/006141**

(87) Internationale Veröffentlichungsnummer:
**WO 2008/009380 (24.01.2008 Gazette 2008/04)**

(54) **VERFAHREN UND VORRICHTUNG ZUR VORGABE VON BEHANDLUNGSPARAMETERN FÜR EXTRAKORPORALE DIALYSEBEHANDLUNG**

METHOD AND DEVICE FOR PREDETERMINING TREATMENT PARAMETERS FOR EXTRACORPORAL DIALYSIS TREATMENT

PROCÉDÉ ET DISPOSITIF DE PRÉDÉTERMINATION DE PARAMÈTRES DE TRAITEMENT POUR UN TRAITEMENT PAR DIALYSE EXTRACORPORELLE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **15.07.2006 DE 102006032926**

(43) Veröffentlichungstag der Anmeldung:
**01.04.2009 Patentblatt 2009/14**

(73) Patentinhaber: Fresenius Medical Care
**Deutschland GmbH**
**61352 Bad Homburg v.d.H. (DE)**

(72) Erfinder: HILGERS, Peter
**97453 Schonungen (DE)**

(74) Vertreter: Oppermann, Frank et al
**OANDO Oppermann & Oppermann LLP**
**John-F.-Kennedy-Straße 4**
**65189 Wiesbaden (DE)**

(56) Entgegenhaltungen:
**EP-A- 1 062 960     WO-A-00/38761**

## Beschreibung

Die Erfindung bezieht sich auf eine Vorrichtung und ein Verfahren zur Vorgabe von Behandlungsparametern für eine extrakorporale Dialysebehandlung mit einem Dialysator, der durch eine semipermeable Membran in eine Blutkammer und eine Dialysierflüssigkeitskammer unterteilt ist, wobei die Blutkammer von Blut mit einer vorgegebenen Blutflussrate und die Dialysierflüssigkeitskammer von Dialysierflüssigkeit mit einer vorgegebenen Dialysierflüssigkeitsrate durchströmt wird. Darüber hinaus betrifft die Erfindung eine Vorrichtung zur extrakorporalen Dialysebehandlung sowie ein Computerprogrammprodukt.

Der Stoffaustausch im Dialysator hat sowohl konvektiven als auch diffusiven Charakter. Beim diffusiven Stoffaustausch ist für die betreffende Substanz der Massentransfer pro Zeiteinheit über die Membran proportional dem Konzentrationsgradienten zwischen Blutplasma und Dialysierflüssigkeit, während beim konvektiven Stofftransport der Massentransfer von der Filtratmenge abhängt, da die Konzentration filtrierbarer Substanzen sowohl im Blutplasma als auch im Filtrat gleich ist.

Da sich das Konzentrationsgefälle während der Dialysebehandlung ständig verringert, kann für die pro Zeiteinheit ausgetauschte Substanzmenge kein fester Zahlenwert angegeben werden. Eine konzentrationsunabhängige Messgröße für die Leistungsfähigkeit eines Dialysators stellt die Clearance K dar.

Die Clearance K einer Substanz ist der Teilstrom des Gesamtstroms durch den Dialysator, der von der betreffenden Substanz vollständig befreit wird. Ein weiterer Begriff zur Bestimmung der Leistungsfähigkeit eines Dialysators ist die Dialysanz D bei der auch die Konzentration der Substanz in der Dialysierflüssigkeit Berücksichtigung findet.

Für die Effektivität einer Dialysebehandlung ist von entscheidender Bedeutung die sogenannte Dialysedosis K*T/V, die als der Quotient aus dem Produkt von Clearance K für Harnstoff und effektiver Behandlungszeit T der Dialysebehandlung und dem Verteilungsvolumen V des Patienten für Harnstoff definiert ist. Nach heutigem medizinischen Kenntnisstand wird gefordert, dass bei einer Dialysebehandlung der Mindestwert der Dialysedosis KT/V bei 1,2 (DOQI National Kidney Foundation, USA) oder 1,4 (European Best Practice Guidelines) liegen sollte. Um sicher zu stellen, dass bei einer Dialysebehandlung eine bestimmte Dialysedosis nicht unterstritten wird, ist allgemeiner Kenntnisstand, die tatsächlich verabreichte Dialysedosis in regelmäßigen Abständen zu überprüften. In der Praxis wird die Dialysedosis im Allgemeinen monatlich überprüft.

Es sind verschiedene Verfahren zur Bestimmung der Dialysedosis KT/V bekannt. Während die Behandlungszeit T als bekannt vorausgesetzt werden kann, müssen die Clearance K und das Verteilungsvolumen V erst bestimmt werden, wenn der Quotient KT/V durch die einzelnen Größen bestimmt werden soll. Hierzu sind verschiedene Verfahren bekannt.

Zunächst kann die Clearance K aus den nominalen Leistungsparametern des Dialysators bestimmt werden. Beispielsweise kann die Clearance für verschiedene Typen von Dialysatoren in Abhängigkeit von der Blutflussrate und der Dialysierflüssigkeitsrate in Form einer Tabelle bereitgestellt werden. Es ist aber auch möglich, die Clearance während der Dialysebehandlung online zu messen. Zur Online-Messung der Clearance K sind wiederum verschiedene Verfahren bekannt. Eines dieser Verfahren beruht darauf, die Leitfähigkeit der Dialysierflüssigkeit stromauf des Dialysators kurzzeitig zu erhöhen und die Leitfähigkeit der Dialysierflüssigkeit stromab des Dialysators zu messen.

Die EP 0 428 927 A1 beschreibt ein Verfahren zur Bestimmung der Dialysanz, bei dem der Dialysat-Elektrolyttransfer jeweils bei zwei unterschiedlichen Dialysateingangskonzentrationen gemessen wird. Unter der Annahme, dass die Bluteingangskonzentration konstant ist, wird nach dem bekannten Verfahren die Dialysanz dadurch bestimmt, dass die Differenz zwischen den Differenzen der Dialysierflüssigkeits-Ionenkonzentration an der Eingangsseite und der Ausgangsseite des Dialysators zum Zeitpunkt der ersten und zweiten Messung ermittelt wird, diese durch die Differenz der Dialysierflüssigkeits-Ionenkonzentration an der Eingangsseite zum Zeitpunkt der ersten Messung und der zweiten Messung geteilt wird und mit dem Dialysierflüssigkeitsfluss multipliziert wird.

Aus der EP 1 062 960 A2 ist ein Verfahren bekannt, bei dem die Dialysanz oder Clearance für eine vorgegebene Dialysierflüssigkeits- und/oder Blutflussrate gemessen und dann die Dialysanz oder Clearance für beliebige Dialysierflüssigkeits- und Blutflussraten berechnet wird.

Das Patientenvolumen V kann aus anthropometrischen Schätzformeln abhängig von Geschlecht, Körpergröße und Alter etc. des Patienten berechnet werden.

Nachdem Zähler und Nenner des Quotienten KT/V bekannt sind, kann schließlich die Dialysedosis berechnet werden.

Der entscheidende Nachteil dieser Methode liegt darin, dass die anthropometrischen Schätzformeln für Gesunde entwickelt worden sind und für Dialysepatienten zu erheblichen Fehlern führen können (Kloppenburg W, Stegemann C et al: Anthropometrybased equations overestimate the urea distribution volume in hemodialysis patients. Kidney Int. 2001; 59: 1165-1174). Aus diesem Grund wird diese Methode in den genannten europäischen und amerikanischen Empfehlungen auch nicht berücksichtigt.

Ein alternatives Verfahren zur Bestimmung der Dialysedosis beruht darauf, dem Patienten prä- und postdialytische Blutproben zu entnehmen und daraus die Harnstoffkonzentrationen zu bestimmen. Hierzu werden prädialytische Proben von ein oder zwei Wochentagen benötigt sowie eine postdialytische Probe. Die Clearance K wird wiederum aus den nominalen Leistungsparametern des Dialysators berechnet oder mit dem bekannten Verfahren online während der Dialysebehandlung gemessen.

In einem komplexen, iterativen Rechenverfahren können dann aus der Clearance K und den Konzentrationen das gesuchte Patientenvolumen V und die Harnstoffgenerationsrate G gemeinsam ermittelt werden. Der Nachteil dieser Methode liegt in der Komplexität des Rechenvorgangs, der ohne Computerunterstützung nicht durchführbar ist. Weiterhin besteht die Gefahr, dass die schwer durchschaubare, abstrakte mathematische Vorgehensweise zu einer relativ geringen Akzeptanz führen könnte.

Eine weitere Alternative zur Bestimmung der Dialysedosis mit einem verhältnismäßig geringen Aufwand ist die Anwendung bekannter Schätzformeln. Diese erlauben, aus Harnstoffkonzentrationen und wenigen, leicht zu ermittelnden Größen die Dialysedosis KT/V zu bestimmen, ohne jedoch die Clearance K und das Patientenvolumen Vermitteln zu müssen. Die bekannteste Schätzformel ist die sogenannte zweite Generationsformel von Daugirdas (Daugirdas, J: Second generation logarithmic estimates of single-pool variable volume kt/v: an analysis of error. J. Am. Soc. Nephrol. 1993; 4: 1205-1213). Nach dieser Formel berechnet sich die Dialysedosis KT/V wie folgt:

$$K*T/V = -\ln (BUN_{post}/BUN_{pre} - 0.008*T) + (4 - 3{,}5*BUN_{post}/BUN_{pre})*(UF/W),$$

wobei ist:

BUN pre: prädialytische Harnstoffkonzentraiton in mmol/l
BUN post: postdialytische Hamstoffkonzentration in mol/l
T: Dialysezeit in h
UF: Ultrafiltrationsvolumen in 1
W: postdialytisches Gewicht in kg.

Mit der obigen Schätzformel kann die Dialysedosis üblicherweise mit einem Fehler von weniger als 5 % bestimmt werden.

Mit den oben beschriebenen Methoden kann zwar die Dialysedosis einer Dialysebehandlung bestimmt werden, der Nachteil der bekannten Verfahren liegt aber darin, dass aus dem Ergebnis nicht in einfacher Weise eine Handlungsempfehlung für den behandelnden Arzt folgen kann. Wenn beispielsweise bei einem Patienten ein Ist-Wert für die Dialysedosis $(KT/V)_{ist}$ von 1,15 festgestellt wird, aber eine Soll-Dialysedosis $(KT/V)_{neu}$ von 1 angestrebt wird, bleibt offen, wie die Dialysevorrichtung zu betreiben ist.

Der Erfindung liegt daher die Aufgabe zugrunde, ein Verfahren zur Vorgabe von Behandlungsparametern für eine extrakorporale Dialysebehandlung anzugeben, das die Ermittlung der vom behandelnden Arzt vorzugebenden Clearance K, mit der die Dialysevorrichtung betrieben werden soll, auf einfache Weise erlaubt, wenn die Clearance K für eine vorausgehende Dialysebehandlung als Ist-Wert bekannt ist und für eine nachfolgende Dialysebehandlung ein neuer Soll-Wert für die Dialysedosis vorgegeben werden soll.

Darüber hinaus ist eine Aufgabe der Erfindung, eine Vorrichtung zur Vorgabe von Behandlungsparametern für eine extrakorporale Dialysebehandlung bereitzustellen, mit der die Ermittlung der Clearance K für eine nachfolgende Dialysebehandlung ohne großen Aufwand möglich ist, wenn für die Dialysedosis ein neuer Soll-Wert vorgegeben wird.

Eine weitere Aufgabe der Erfindung ist, ein Verfahren und eine Vorrichtung anzugeben, um die neue Behandlungszeit für eine nachfolgende Dialysebehandlung für eine bestimmte Clearance der Dialysebehandlung auf einfache Weise zu ermitteln. Dieser Fall ist aber weniger praxisnah als der obige Fall, der die Vorgabe der Clearance für eine bestimmte Behandlungszeit vorsieht.

Weiterhin ist eine Aufgabe der Erfindung eine Vorrichtung zur extrakorporalen Dialysebehandlung und ein Computerprogrammprodukt anzugeben, um ohne großen Aufwand dem Arzt die Möglichkeit zu geben, eine Dialysevorrichtung derart zu betreiben, dass mit der Dialysebehandlung eine bestimmte Dialysedosis erzielt wird.

Die Lösung dieser Aufgaben erfolgt erfindungsgemäß mit den Merkmalen der Patentansprüche 1, 6, 7, 11 und 13. Vorteilhafte Ausführungsformen der Erfindung sind Gegenstand der Unteransprüche.

Das erfindungsgemäße Verfahren und die erfindungsgemäße Vorrichtung zur Vorgabe von Behandlungsparametern sowie die Dialysevorrichtung beruhen darauf, dass der Ist-Wert der Dialysedosis sowie der Clearance sowie der Behandlungszeit einer Dialysebehandlung bestimmt und ein Soll-Wert der Dialysedosis für eine nachfolgende Dialysebehandlung mit einer bestimmten Behandlungszeit vorgegeben wird. Dabei ist die im Allgemeinen vorgegebene Behandlungszeit ohnehin bekannt. Aus den Ist-Werten der Dialysedosis, der Clearance und der Behandlungszeit und dem Soll-Wert der Dialysedosis wird dann die Soll-Clearance für die nachfolgende Behandlung bei Vorgabe der Behandlungszeit berechnet. Umgekehrt kann aber grundsätzlich auch die Behandlungszeit bei Vorgabe einer bestimmten Clearance ermittelt werden. Die Berechnung von Clearance oder Behandlungszeit der nachfolgenden Behandlung kann erfolgen, wenn die Clearance und Behandlungszeit der vorausgehenden Behandlung bekannt ist. Grundsätzlich ist es aber ausreichend, wenn nicht die Clearance und Behandlunsgzeit als solche, sondern nur das Produkt von Clearance und Behandlunsgzeit der vorausgehenden Behandlung bekannt sind.

Der neue Wert für die Clearance kann auf einer Anzeigeeinheit angezeigt werden, so dass die Parameter der Dialysevorrichtung, von denen die Clearance abhängig ist, entsprechend eingestellt werden können. Es ist aber auch möglich, dass nach Berechnung des neuen Wertes für die Clearance die relevanten Dialyseparameter, zu denen der Blutfluss und der Dialysierflüssigkeitsfluss sowie die Behandlungszeit zählen, von der Dialysevorrichtung mit der neuen Zielvorgabe automatisch eingestellt werden.

Ein entscheidender Vorteil liegt darin, dass der neue Wert der Clearance bestimmt werden kann, ohne das Verteilungsvolumen bestimmen zu müssen. Daher brauchen die bekannten Verfahren zur Schätzung, Messung oder Berechnung des Verteilungsvolumens nicht angewandt zu werden. Der Arzt kann die Änderung der Therapie mit dem erfindungsgemäßen Verfahren und der erfindungsgemäßen Vorrichtung ohne großen Aufwand in kurzer Zeit vornehmen, wobei mit der Bestimmung eines Ist-Wertes und der Vorgabe eines Soll-Wertes der Dialysedosis als einzig relevante Größen neben dem Ist-Wert der Clearance und der ohnehin vorgegebenen Behandlungszeit für den Arzt eine große Transparenz bei Therapieänderungen gegeben ist.

Das erfindungsgemäße Verfahren und die erfindungsgemäße Vorrichtung erlauben mit der Kontrolle des Ist-Wertes der Dialysedosis in festgelegten Zeitabständen, beispielsweise in einem Monat, und der manuellen oder automatischen Vorgabe eines neuen Soll-Wertes für die Dialysedosis nach der Bewertung des Ist-Wertes eine Optimierung der Dialysebehandlung. Die Bewertung des Ist-Wertes der Dialysedosis kann nach den entsprechenden Richtlinien (European Best Practice Guidelines oder DOQI, National Kidney Foundation, USA) einfach durch Vergleich des Ist-Wertes mit einem Zielwert, beispielsweise 1,4 oder 1,2 erfolgen, wobei für die nachfolgende Behandlung eine größere Dialysedosis vorgegeben wird, wenn die Dialysedosis für die vorausgehende Behandlung den vorgegebenen Mindestwert unterschreiten sollte.

Für die Berechnung des neuen Wertes der Clearance ist neben dem Ist-Wert der Dialysedosis für die vorausgehende Dialysebehandlung noch die Clearance der vorausgehenden Dialysebehandlung zu bestimmen. Die Clearance der vorausgehenden Dialysebehandlung kann auf einfache Weise durch die Leistungsparameter des Dialysators bestimmt werden. Beispielsweise können in Abhängigkeit von unterschiedlichen Typen von Dialysatoren die unterschiedlichen Werte für die Clearance in einem Speicher oder einer Tabelle abgelegt werden.

Eine bevorzugte Ausführungsform der Erfindung sieht jedoch vor, die Clearance der vorausgehenden Dialysebehandlung während der Behandlung zu messen. Zur Online-Messung der Clearance während der Behandlung sind dem Fachmann Verfahren bekannt.

Die erfindungsgemäße Vorrichtung zur Vorgabe von Behandlungsparametern sowie die erfindungsgemäße Dialysevorrichtung verfügen über eine Eingabeeinheit zum Eingeben des Soll-Wertes der Dialysedosis für die nachfolgende Dialysebehandlung und einer bestimmten Behandlungszeit sowie eine Recheneinheit, die derart ausgebildet ist, dass der neue Wert der Clearance aus den Ist-Werten der Dialysedosis, der Clearance und der Behandlungszeit einer vorausgehenden Dialysebehandlung und dem Soll-Wert der Dialysedosis für die nachfolgende Dialysebehandlung und der Behandlungszeit der nachfolgenden Dialysebehandlung berechnet wird. Dabei wird davon ausgegangen, dass die Clearance und die Behandlungszeit der vorausgehenden Behandlung als bekannte Größen nicht mit der Eingabeeinheit eingegeben werden müssen. Wenn dies aber nicht der Fall ist, wird mit der Eingabeeinheit auch die Clearance und/ oder die Behandlungszeit der vorausgehenden Behandlung eingegeben.

Wenn der Ist-Wert der Clearance aus den nominalen Leistungsparametern des Dialysators berechnet wird, verfügt die erfindungsgemäße Vorrichtung weiterhin über eine Speichereinheit zum Speichern der jeweiligen Clearance der Dialysebehandlung für eine Anzahl von Dialysatoren mit unterschiedlichen Leistungsparametern.

Bei einer bevorzugten Ausführungsform jedoch, die eine Messung der Clearance vorsieht, verfügt die erfindungsgemäße Vorrichtung über eine Auswert- und Messeinheit, mit der nach den bekannten Verfahren die Clearance während der Behandlung bestimmt werden kann.

Im folgenden werden unter Bezugnahme auf die Zeichnungen zwei Ausführungsbeispiele der Erfindung näher erläutert.

Es zeigen:

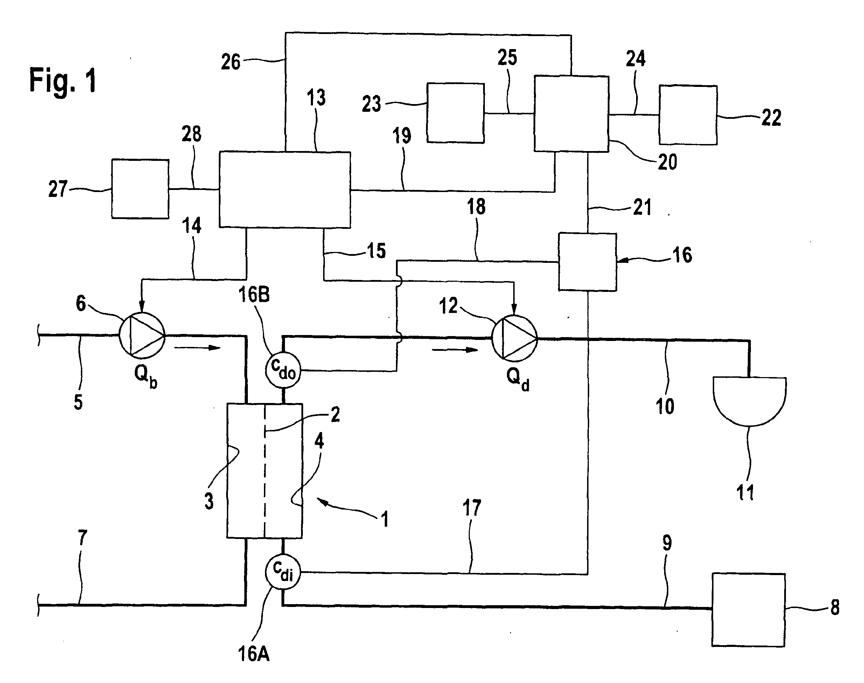

Fig. 1    eine stark vereinfachte schematische Darstellung der wesentlichen Komponenten einer Vorrichtung zur extrakorporalen Dialysebehandlung, die über eine Vorrichtung zur Vorgabe von Behandlungsparametern verfügt, wobei nach Vorgabe eines neuen Wertes der Dialysedosis für eine nachfolgende Dialysebehandlung die Dialysevorrichtung den neuen Wert der Clearance für die nachfolgende Dialysebehandlung automatisch als Behandlungsparameter übernimmt und

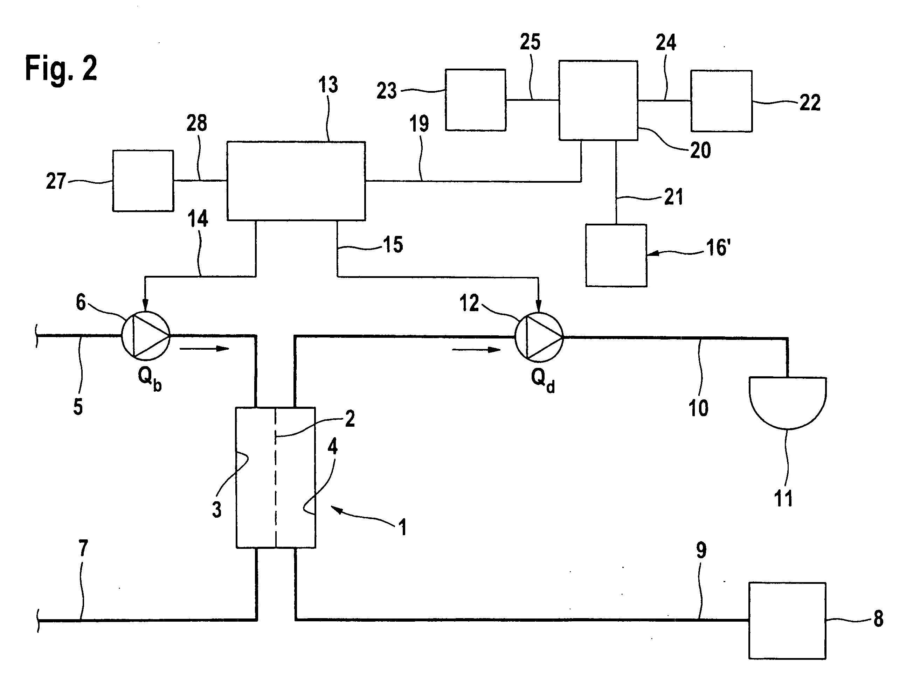

Fig. 2    ein zweites Ausführungsbeispiel der wesentlichen Komponenten einer Vorrichtung zur extrakorporalen Dialysebehandlung, die über eine Vorrichtung zur Vorgabe von Behandlungsparametern verfügt.

Die erfindungsgemäße Vorrichtung zur Vorgabe von Behandlungsparametern kann Teil einer Vorrichtung zur extrakorporalen Dialysebehandlung sein. Dabei kann die erfindungsgemäße Vorrichtung von einzelnen Komponenten Gebrauch machen, die in einer Dialysevorrichtung ohnehin vorhanden sind. Alternativ kann die erfindungsgemäße Vorrichtung aber auch eine zusätzliche Einheit bilden, die separat von der Dialysevorrichtung betrieben wird. In den nachfolgenden Ausführungsbeispielen wird die erfindungsgemäße Vorrichtung als Teil der Dialysevorrichtung beschrieben.

Fig. 1 zeigt ein erstes Ausführungsbeispiel der erfindungsgemäßen Dialysevorrichtung, die über einen Dialysator 1 verfügt, der durch eine semipermeable Membran 2 in eine Blutkammer 3 und eine Dialysierflüssigkeitskammer 4 unterteilt ist. Von einem Patienten führt eine arterielle Blutleitung 5, in die eine Blutpumpe 6 geschaltet ist, zu einem Einlass der Blutkammer 3, während von einem Auslass der Blutkammer eine venöse Blutleitung 7 zu dem Patienten führt.

In einer Dialysierflüssigkeitsquelle 8 wird frische Dialysierflüssigkeit bereitgestellt. Von der Dialysierflüssigkeitsquelle 8 führt eine Dialysierflüssigkeitszuführleitung 9 zu einem Einlass der Dialysierflüssigkeitskammer 4 des Dialysators 1, während eine Dialysierflüssigkeitsabführleitung 10 von einem Auslass der Dialysierflüssigkeitskammer zu einem Abfluss 11 führt. In die Dialysierflüssigkeitsabführleitung 10 ist eine Dialysierflüssigkeitspumpe 12 geschaltet.

Die Dialysevorrichtung verfügt über eine Steuereinheit 13, die mit der Blutpumpe 6 und der Dialysierflüssigkeitspumpe 12 über Steuerleitungen 14, 15 verbunden ist. Die Steuereinheit 13 erzeugt Steuersignale zum Betreiben der Blut- und Dialysierflüssigkeitspumpe 6, 12 mit einer vorgegebenen Förderrate, so dass sich in der Blutleitung 5 eine vorgegebene Blutflussrate $Q_b$ und in der Dialysierflüssigkeitsleitung 10 eine vorgegebene Dialysierflüssigkeitsrate $Q_d$ einstellen.

Darüber hinaus verfügt die Dialysevorrichtung über eine Mess- und Auswerteinheit 16 zur online Bestimmung des Ist-Wertes der Clearance $K_{ist}$ während der Dialysebehandlung. Da eine derartige Mess- und Auswerteinheit zur Bestimmung der Clearance während der Dialysebehandlung bereits zum Stand der Technik gehört, wird deren Aufbau und Funktionsweise nur kurz beschrieben.

Die Mess- und Auswerteinheit 16 weist einen am Einlass der Dialysierflüssigkeitskammer 4 in der Dialysierflüssigkeitszuführleitung 9 angeordneten Leitfähigkeitssensor 16A und einen in der Dialysierflüssigkeitsabführleitung 10 am Auslass der Dialysierflüssigkeitskammer angeordneten Leitfähigkeitssensor 16B auf. Nach Initiierung einer kurzzeitigen Änderung der Leitfähigkeit in der Dialysierflüssigkeit misst der Leitfähigkeitssensor 16A stromauf der Dialysierflüssigkeitskammer 4 die Dialysierflüssigkeitseingangskonzentration $c_{di}$ von Natrium in der Dialysierflüssigkeit und der Leitfähigkeitssensor 16B die Dialysierflüssigkeitsausgangskonzentration $c_{do}$ von Natrium in der Dialysierflüssigkeit stromab der Dialysierflüssigkeitskammer 4 des Dialysators 1.

Die Messwerte der Leitfähigkeitssensoren 16A, 16B werden über Signalleistungen 17, 18 der Mess- und Auswerteinheit 16 zugeführt, die über eine Datenleitung 19 von der Steuereinheit 13 weitere für die Bestimmung der Clearance relevante Größen empfängt, zu denen der Blutfluss $Q_b$ und der Dialysierflüssigkeitsfluss $Q_d$ sowie die Behandlungszeit T zählen. Die Mess- und Auswerteinheit 16 berechnet während der Dialysebehandlung fortlaufend den Ist-Wert der Clearance $K_{ist}$ für Harnstoff, die der aus den Natriumkonzentrationswerten ermittelten Dialysance für Natrium aufgrund des ähnlichen Diffusionsverhaltens entspricht. Die Bestimmung der Dialysance kann beispielsweise erfolgen, wie in der EP 0 428 927 A1 beschrieben ist.

Neben der Mess- und Auswerteinheit 16 umfasst die erfindungsgemäße Vorrichtung zur Vorgabe von Behandlungsparametern eine Recheneinheit 20, die über eine Datenleitung 21 mit der Mess- und Auswerteinheit 16 und eine weitere Datenleitung 26 mit der Steuereinheit 13 verbunden ist sowie eine Eingabeeinheit 22 und eine Anzeigeeinheit 23, die jeweils über Datenleitungen 24, 25 mit der Recheneinheit 20 verbunden sind.

Mit der Eingabeeinheit 22, die beispielsweise als Tastatur oder Bildschirmeingabegerät (Touch-Screen) ausgebildet sein kann, können die Behandlungszeit T sowie die Dialysedosis (KT/V) der Dialysebehandlung eingegeben werden. Neben der Behandlungszeit und der Dialysedosis können noch weitere Behandlungsparameter, beispielsweise der Dialysierflüssigkeitsfluss $Q_d$ und der Blutfluss $Q_b$ eingegeben werden. Hierzu kann aber auch eine weitere Eingabeeinheit vorgesehen sein.

Bei dem Ausführungsbeispiel von Fig. 1 kommuniziert die Recheneinheit 20 mit der Steuereinheit 13 der Dialysevorrichtung über die Datenleitung 26, so dass die Recheneinheit 20 der Steuereinheit 13 Behandlungsparameter vorgeben kann, mit denen die Dialysebehandlung dann durchgeführt wird.

Darüber hinaus verfügt die Dialysevorrichtung über eine Schnittstelle 28, die mit der Steuereinheit 13 über eine Datenleitung 29 verbunden ist. Die Schnittstelle 28 dient zur Kommunikation mit nicht dargestellten externen Einheiten. Über die Schnittstelle 28 können Programme (Software) eingelesen werden, um die Dialysevorrichtung mit den erfindungsgemäßen Routinen betreiben zu können. Die Programme können mit den bekannten Datenträgern bereitgestellt werden

Nachfolgend wird die Funktionsweise der Dialysevorrichtung im einzelnen beschrieben.

Es sei angenommen, dass ein Ist-Wert $(KT/V)_{ist}$ für die Dialysedosis vorliegt, der sich aufgrund von Messungen bei vorausgehenden Dialysebehandlungen ergibt. Unter der Annahme, dass sich das Verteilungsvolumen V des Patienten nicht wesentlich ändert, gilt das Folgende:

$$V_{neu} = V_{ist}$$

$$\Rightarrow \quad K_{neu} T_{neu} V_{neu}/(K_{neu}T_{neu}) = K_{ist}T_{ist}V_{ist}/(K_{ist}T_{ist})$$

$$\Rightarrow \quad K_{neu}T_{neu}/(KT/V)_{neu} = K_{ist}T_{ist}/(KT/V)_{ist}$$

$$\Rightarrow \quad K_{neu}T_{neu} = K_{ist}T_{ist}(KT/V)_{neu}/(KT/V)_{ist}.$$

Mit der Dialysevorrichtung wird unter Vorgabe bestimmter Behandlungsparameter, zu denen der Dialysierflüssigkeitsfluss $Q_d$, der Blutfluss $Q_b$ und die Behandlungszeit $T_{ist}$ zählen, eine Dialysebehandlung durchgeführt. Die Behandlungsparameter können mit der Eingabeeinheit 22 eingegeben werden. Während der Dialysebehandlung bestimmt die Mess- und Auswerteinheit 16 den Ist-Wert der Clearance $K_{ist}$, der auf der Anzeigeeinheit 23 angezeigt wird.

Es sei angenommen, dass der Patient mit der Behandlungszeit $T_{ist}$ und der Clearance $K_{ist}$ behandelt wird. Mit der Dialysebehandlung wird eine Dialysedosis erzielt, die nach der Behandlung bestimmt wird. Die Bestimmung des Ist-Wertes der Dialysedosis $(KT/V)_{ist}$ kann auch beispielsweise nach der eingangs beschriebenen Schätzformel nach Daugirdas erfolgen.

Wenn sich herausstellt, dass der Ist-Wert der Dialysedosis kleiner als ein vorgegebener Zielwert, beispielsweise 1,2 oder 1,4 ist, entscheidet der Arzt, ob die Behandlungsparameter geändert werden.

Die erfindungsgemäße Vorrichtung erlaubt es dem Arzt, für die nachfolgende Dialysebehandlung lediglich einen neuen Zielwert für die Dialysedosis $(KT/V)_{neu}$ vorzugeben, wobei die nachfolgende Dialysebehandlung dann automatisch so durchgeführt wird, dass diese Dialysedosis erzielt wird.

Der Arzt gibt mit der Eingabeeinheit 22 lediglich den Ist-Wert der Dialysedosis $(KT/V)_{ist}$ für die vorausgehende Dialysebehandlung und den Soll-Wert für die Dialysedosis $(KT/V)_{neu}$ für die nachfolgende Dialysebehandlung ein. Weiterhin gibt der Arzt für die nachfolgende Dialysebehandlung die Behandlungszeit $T_{neu}$ ein. Die Eingabe der Behandlungszeit $T_{ist}$ für die vorausgehende Behandlungszeit erübrigt sich, da der Arzt diesen Wert bereits für die Durchführung der vorausgehenden Dialysebehandlung mit der Eingabeeinheit eingegeben hat. Weiterhin ist der Ist-Wert der Clearance $K_{ist}$ noch in der Recheneinheit 20 gespeichert. Alternativ können die Werte für $T_{ist}$ und $K_{ist}$ auch über die Eingabeeinheit 22 eingegeben werden.

Die Recheneinheit 20 erhält für die Berechnung der Clearance $K_{neu}$ für die nachfolgende Dialysebehandlung von der Eingabeeinheit 22 und der Mess- und Auswerteinheit 16 die Behandlungszeiten $T_{neu}$. $T_{ist}$ für die nachfolgende und die vorausgehende Dialysebehandlung und die Dialysedosen $(KT/V)_{neu}$, $(KTN)_{ist}$ für die nachfolgende und vorausgehende Dialysebehandlung und den Wert der Clearance $K_{ist}$ für die vorausgehende Dialysebehandlung. Aus diesen Größen berechnet die Recheneinheit 20 den Wert der Clearance $K_{neu}$ für die nachfolgende Dialysebahndlung nach der folgenden Gleichung:

$$K_{neu}*T_{neu} = K_{ist}*T_{ist}*(K*T/V)_{neu}/(K*T/V)_{ist}$$

Beispiel: $T_{ist} = T_{neu} = 4h$

$(K*T/V)_{ist} = 1.15$
$(K*T/V)_{neu} = 1.4$
$K_{ist} = 200$ ml/min
Daraus folgt:
$K_{neu} = 200*1.4/1.15$ ml/min $= 243$ ml/min.

Der neue Wert für die Clearance $K_{neu}$, mit dem die nachfolgende Dialysebehandlung durchgeführt werden soll, wird mit der Anzeigeeinheit 23 angezeigt. Der Arzt kann nach Kenntnis dieses Wertes somit die Behandlungsparameter, beispielsweise den Dialysierflüssigkeitsfluss und den Blutfluss vorgeben und einstellen, um die berechnete Clearance zu erzielen. Den Zusammenhang zwischen der Clearance und den einzelnen Behandlungsparametern kann der Arzt beispielsweise Tabellen entnehmen.

Als alternative Ausführungsform, die aber weniger praxisnah ist, kann aber auch die Behandlungszeit $T_{neu}$ für eine bestimmte Clearance $K_{neu}$ vorgegeben werden. Hierzu ist die obige Gleichung nicht nach $K_{neu}$, sondern $T_{neu}$

aufzulösen.

Die erfindungsgemäße Dialysevorrichtung sieht darüber hinaus eine automatische Steuerung der Dialysemaschine derart vor, dass die Recheneinheit 20 der Steuereinheit 13 nach der Berechnung des neuen Wertes für die Clearance $K_{neu}$ die Behandlungsparameter automatisch vorgibt, mit denen die neue Clearance bei der vorgegebenen Behandlungszeit $T_{neu}$ erzielt wird. Bei einem vorgegebenen Blutfluss $Q_b$ beispielsweise berechnet die Recheneinheit 20 den Dialysierflüssigkeitsfluss $Q_d$, der erforderlich ist, um die Clearance $K_{neu}$ zu erzielen. Alternativ berechnet die Recheneinheit bei einem vorgegebenen Dialysierflüssigkeitsfluss $Q_d$ den Blutfluss $Q_b$, um die Clearance $K_{neu}$ zu erzielen. Die Steuereinheit 13 betreibt die Dialysevorrichtung dann mit dem vorgegebenen Dialysierflüssigkeits- und Blutfluss sowie der Behandlungszeit, so dass die berechnete Clearance $K_{neu}$ erreicht wird. Nach dieser Dialysebehandlung oder mehreren weiteren Dialysebehandlungen kann dann wieder der Ist-Wert der Dialysedosis bestimmt werden, woraufhin wieder für die nachfolgende oder die nachfolgenden Dialysebehandlungen ein neuer Wert für die Clearance $K_{neu}$ berechnet wird.

Die Steuereinheit 13 ist bei der erfindungsgemäßen Dialysevorrichtung derart ausgebildet, dass nach der Berechnung eines neuen Wertes für die Clearance $K_{neu}$ die Flussraten, mit denen die Behandlung durchgeführt wird, so eingestellt werden, dass der neue Wert für die Clearance $K_{neu}$ beibehalten wird. Beispielsweise ist die Steuereinheit derart ausgebildet, dass nach der Berechnung der Clearance $K_{neu}$ für die nachfolgende Dialysebehandlung bei einer vorgegebenen Blutflussrate $Q_b$ die Dialysierflüssigkeitsrate $Q_d$ berechnet wird, bei der die für die nachfolgende Dialysebehandlung vorgegebene Clearance $K_{neu}$ beibehalten wird. Alternativ kann auch bei einer vorgegebenen Dialysierflüssigkeitsrate $Q_d$ die Blutflussrate $Q_b$ berechnet werden, bei der die neue Clearance $K_{neu}$ beibehalten wird. Die Berechnung der Flussraten erfolgt allein auf der Grundlage einer vorgegebenen Abhängigkeit der Clearance von den Flussraten.

Die Berechnung der jeweiligen Flussraten zur Beibehaltung der Clearance, zu denen neben der Dialysierflüssigkeitsrate und Blutflussrate auch die Ultrafiltratrate und Substituatrate zählen können, ist in der deutschen Patentanmeldung vom 08. Juni 2006 mit dem Aktenzeichen 10 2006 026 999.3 oder der deutschen Patentanmeldung vom 17. August 2006 mit dem Aktenzeichen 10 2006 038 545.4, die die Priorität der obigen Patentanmeldung in Anspruch nimmt, im Einzelnen ausführlich beschrieben. Daher wird zum Zwecke der Offenbarung auf diese Druckschrift ausdrücklich Bezug genommen.

Fig. 2 zeigt ein zweites Ausführungsbeispiel der erfindungsgemäßen Dialysevorrichtung, das sich von dem unter Bezugnahme auf Fig. 1 beschriebenen Ausführungsbeispiel zum einen dadurch unterscheidet, dass die nachfolgende Dialysebehandlung nicht automatisch so durchgeführt wird, dass der neue Wert für die Clearance $K_{neu}$ erzielt wird. Nach diesem Ausführungsbeispiel wird der neue Wert für die Clearance lediglich mit der Anzeigeeinheit 23 angezeigt, so dass der Arzt die erforderlichen Behandlungsparameter selbst vorgibt, beispielsweise den Blut- und Dialysierflüssigkeitsfluss derart einstellt, dass die Clearance erzielt wird. Bei diesem Ausführungsbeispiel entfällt somit die Datenleitung 26, mit der die Recheneinheit 20 mit der Steuereinheit 13 kommuniziert. Zum anderen unterscheidet sich das zweite Ausführungsbeispiel von der ersten Ausführungsform dadurch, dass die Clearance $K_{ist}$ für die vorausgehende Dialysebehandlung nicht gemessen, sondern aus den nominalen Leistungsparametern des Dialysators bestimmt wird. Damit entfällt die Mess- und Auswerteinheit 16 mit den zugehörigen Leitfähigkeitssensoren 16A, 16B, welche durch eine Speichereinheit 16' ersetzt wird, die in Form einer Tabelle für unterschiedliche Typen von Dialysatoren die entsprechenden Werte der Clearance für die einzelnen Blutflüsse $Q_b$ und Dialysierflüssigkeitsflüsse $Q_d$ enthält. Nach Vorgabe eines bestimmten Dialysatortyps liest die Recheneinheit 20 über die Datenleitung 21 dann den entsprechenden Wert für die Clearance $K_{ist}$ aus. Die übrigen Komponenten des zweiten Ausführungsbeispiels sind mit denen der ersten Ausführungsform identisch. Daher werden auch für dieselben Komponenten dieselben Bezugszeichen verwendet. Es ist auch möglich, einzelne Aspekte der unterschiedlichen Ausführungsformen miteinander zu kombinieren, ohne vom Konzept der Erfindung abzusehen.

**Patentansprüche**

1. Verfahren zur Vorgabe von Behandlungsparametern für eine extrakorporale Dialysebehandlung mit einem Dialysator, der durch eine semipermeable Membran in eine Blutkammer und eine Dialysierflüssigkeitskammer unterteilt ist, wobei die Blutkammer von Blut mit einer vorgegebenen Blutflussrate $Q_b$ und die Dialysierflüssigkeitskammer von Dialysierflüssigkeit mit einer vorgegebenen Dialysierflüssigkeitsrate $Q_d$ durchströmt wird, mit folgenden Verfahrensschritten:

   Bestimmen des Ist-Wertes der Dialysedosis einer Dialysebehandlung, wobei die Dialysedosis $(KT/V)_{ist}$ dem Produkt von Clearance K und Behandlungszeit T der Dialysebehandlung geteilt durch das Verteilungsvolumen V des Patienten entspricht,
   Bestimmen der Clearance $K_{ist}$ und der Behandlungszeit $T_{ist}$ der Dialysebehandlung oder des Produktes von

Clearance $K_{ist}$ und Behandlungszeit $T_{ist}$,

Vorgabe eines Soll-Wertes der Dialysedosis $(KT/V)_{neu}$ für eine nachfolgende Dialysebehandlung und einer bestimmten Behandlungszeit $T_{neu}$ oder einer bestimmten Clearance $K_{neu}$ für die nachfolgende Dialysebehandlung,

Berechnen der Clearance $K_{neu}$ aus dem Ist-Wert der Dialysedosis $(KT/V)_{ist}$ der vorausgehenden Dialysebehandlung und dem Soll-Wert der Dialysedosis $(KT/V)_{neu}$ für die nachfolgende Dialysebehandlung und der Behandlungszeit $T_{neu}$ der nachfolgenden Dialysebehandlung und der Clearance $K_{ist}$ und der Behandlungszeit $T_{ist}$ der vorausgehenden Dialysebehandlung oder dem Produkt von Clearance $K_{ist}$ und Behandlungszeit $T_{ist}$, und Vorgabe der Clearance $K_{neu}$ als neuen Behandlungsparameter und/oder

Berechnen der Behandlungszeit $T_{neu}$ aus dem Ist-Wert der Dialysedosis $(KT/V)_{ist}$ der vorausgehenden Dialysebehandlung und dem Soll-Wert der Dialysedosis $(KT/V)_{neu}$ für die nachfolgende Dialysebehandlung und der Clearance $K_{neu}$ der nachfolgenden Dialysebehandlung und der Clearance $K_{ist}$ und der Behandlungszeit $T_{ist}$ der vorausgehenden Dialysebehandlung oder dem Produkt von Clearance $K_{ist}$ und Behandlungszeit $T_{ist}$, und Vorgabe der Behandlungszeit $T_{neu}$ als neuen Behandlungsparameter.

2. Verfahren nach Anspruch 1, **dadurch gekennzeichnet, dass** die Clearance $K_{neu}$ aus dem Ist-Wert der Dialysedosis $(KT/V)_{ist}$ der vorausgehenden Dialysebehandlung und dem Soll-Wert der Dialysedosis $(KT/V)_{neu}$ für die nachfolgende Dialysebehandlung und der Behandlungszeit $T_{neu}$ der nachfolgenden Dialysebehandlung nach der folgenden Gleichung berechnet wird:

$$K_{neu} = K_{ist} T_{ist} / T_{neu} \, [(KT/V)_{neu} / (KT/V)_{ist}]$$

3. Verfahren nach Anspruch 1 oder 2, **dadurch gekennzeichnet, dass** die Clearance $K_{ist}$ der vorausgehenden Dialysebehandlung aus den Leistungsparametern des Dialysators bestimmt wird.

4. Verfahren nach Anspruch 1 oder 2, **dadurch gekennzeichnet, dass** die Clearance $K_{ist}$ der vorausgehenden Dialysebehandlung während der Dialysebehandlung gemessen wird.

5. Verfahren nach einem der Ansprüche 1 bis 4, **dadurch gekennzeichnet, dass** der Ist-Wert der Dialysedosis $(KT/V)_{ist}$ der vorausgehenden Dialysebehandlung nach der folgenden Gleichung berechnet wird:

$$K*T/V = -\ln (BUN_{post}/BUN_{pre} - 0.008*T) + (4 - 3.5*BUN_{post}/BUN_{pre})*(UF/W),$$

wobei ist: BUN pre: prädialytische Harnstoffkonzentraiton in mmol/l

BUN post: postdialytische Harnstofikonzentration in mmol/l T: Dialysezeit in h
UF: Ultrafiltrationsvolumen in 1
W: postdialytisches Gewicht in kg.

6. Verfahren zur Einstellung von Behandlungsparamtern vor Durchführung einer extrakorporale Dialysebehandlung mit einem Dialysator, der durch eine semipermeable Membran in eine Blutkammer und eine Dialysierflüssigkeitskammer unterteilt ist, wobei die Blutkammer von Blut mit einer vorgegebenen Blutflussrate $Q_b$ und die Dialysierflüssigkeitskammer von Dialysierflüssigkeit mit einer vorgegebenen Dialysierflüssigkeitsrate $Q_d$ durchströmt wird, **dadurch gekennzeichnet, dass** die Clearance $K_{neu}$ als neuer Behandlungsparameter nach dem Verfahren nach einem der Ansprüche 1 bis 5 eingestellt wird.

7. Vorrichtung zur Vorgabe von Behandlungsparametern für eine extrakorporale Dialysebehandlung mit einem extrakorporalen Blutkreislauf, der einen durch eine semipermeable Membran (2) in eine Blutkammer (3) und eine Dialysierflüssigkeitskammer (4) unterteilten Dialysator (1) enthält, wobei die Blutkammer von Blut mit einer vorgegebenen Blutflussrate $Q_b$ und die Dialysierflüssigkeitskammer von Dialysierflüssigkeit mit einer vorgegebenen Dialysierflüssigkeitsrate $Q_d$ durchströmt wird,
**dadurch gekennzeichnet, dass** die Vorrichtung aufweist:

eine Eingabeeinheit (22) zum Eingeben eines Ist-Wertes der Dialysedosis $(KT/V)_{ist}$ einer vorausgehenden Dialysebehandlung und eines Soll-Wertes der Dialysedosis $(KT/V)_{neu}$ für eine nachfolgende Dialysebehandlung und einer bestimmten Behandlungszeit $T_{neu}$ oder einer bestimmten Clearance $K_{neu}$ für die nachfolgende Dialysebehandlung und

eine Recheneinheit, die derart ausgebildet ist, dass die Clearance $K_{neu}$ aus dem Ist-Wert der Dialysedosis $(KT/V)_{ist}$ der vorausgehenden Dialysebehandlung und dem Soll-Wert der Dialysedosis $(KT/V)_{neu}$ für die nachfolgende Dialysebehandlung und der Behandlungszeit $T_{neu}$ der nachfolgenden Dialysebehandlung und der Clearance $K_{ist}$ und der Behandlungszeit $T_{ist}$ der vorausgehenden Dialysebehandlung oder dem Produkt von Clearance $K_{ist}$ und Behandlungszeit $T_{ist}$ berechnet wird und/oder derart ausgebildet ist, dass die Behandlungszeit $T_{neu}$ aus dem Ist-Wert der Dialysedosis $(KT/V)_{ist}$ der vorausgehenden Dialysebehandlung und dem Soll-Wert der Dialysedosis $(KT/V)_{neu}$ für die nachfolgende Dialysebehandlung und der Clearance $K_{neu}$ der nachfolgenden Dialysebehandlung und der Clearance $K_{ist}$ und der Behandlungszeit $T_{ist}$ der vorausgehenden Dialysebehandlung oder dem Produkt von Clearance $K_{ist}$ und Behandlungszeit $T_{ist}$, berechnet wird.

**8.** Vorrichtung nach Anspruch 7, **dadurch gekennzeichnet, dass** die Recheneinheit (20) derart ausgebildet ist, dass die Clearance $K_{neu}$ aus dem Ist-Wert der Dialysedosis $(KT/V)_{ist}$ der vorausgehenden Dialysebehandlung und dem Soll-Wert der Dialysedosis $(KT/V)_{neu}$ für die nachfolgende Dialysebehandlung und der Behandlungszeit $T_{neu}$ für die nachfolgende Dialysebehandlung nach der folgenden Gleichung berechnet wird:

$$K_{neu} = K_{ist}T_{ist}/T_{neu} \; [(KT/V)_{neu}/(KT/V)_{ist}]$$

**9.** Vorrichtung nach Anspruch 7 oder 8, **dadurch gekennzeichnet, dass** die Vorrichtung ferner eine Speichereinheit (16') zum Speichern der jeweiligen Clearance $K_{ist}$ der Dialysebehandlung für eine Anzahl von Dialysatoren mit unterschiedlichen Leistungsparametern aufweist.

**10.** Vorrichtung nach Anspruch 7 oder 8, **dadurch gekennzeichnet, dass** die Vorrichtung ferner eine Mess- und Auswerteinheit (16) zum Messen der Clearance $K_{ist}$ der vorausgehenden Dialysebehandlung aufweist.

**11.** Vorrichtung zur extrakorporalen Dialysebehandlung mit einem Dialysator (1), der durch eine semipermeable Membran (2) in eine Blutkammer (3) und eine Dialyseflüssigkeitskammer (4) unterteilt ist, wobei die Blutkammer von Blut mit einer vorgegebenen Blutflussrate $Q_b$ und die Dialysierflüssigkeitskammer von Dialysierflüssigkeit mit einer vorgegebenen Dialysierflüssigkeitsrate $Q_d$ durchströmt wird, **dadurch gekennzeichnet, dass** die Dialysevorrichtung eine Vorrichtung nach einem der Ansprüche 7 bis 10 aufweist.

**12.** Vorrichtung zur extrakorporalen Dialysebehandlung nach Anspruch 11, **dadurch gekennzeichnet, dass** die Steuereinheit (13) derart ausgebildet ist, dass nach der Berechnung der Clearance $K_{neu}$ für die nachfolgende Dialysebehandlung bei vorgegebener Blutflussrate $Q_b$ die Dialysierflüssigkeitsrate $Q_d$ oder bei vorgegebener Dialysierflüssigkeitsrate $Q_d$ die Blutflussrate $Q_b$ allein auf der Grundlage einer vorgegebenen Abhängigkeit der Clearance von der Dialysierflüssigkeitsrate und der Blutflussrate berechnet wird, bei der die für die nachfolgende Dialysebehandlung vorgegebene Clearance beibehalten wird.

**13.** Computerprogrammprodukt mit einer Vielzahl von Instruktionen zum Betreiben eines Computers derart, dass die Verfahrensschritte nach einem der Ansprüche 1 bis 6 durchgeführt werden.

**Claims**

**1.** A method for predetermining treatment parameters for an extra-corporal dialysis treatment with a dialyser that is subdivided by a semipermeable membrane into a blood chamber and a dialysis liquid chamber, wherein blood flows at a predetermined blood flow rate $Q_b$ through the blood chamber, and dialysis liquid flows at a predetermined dialysis flow rate $Q_d$ through the dialysis liquid chamber, the method comprising the following steps:

determining the actual value of the dialysis dose of a dialysis treatment, wherein the dialysis dose $(KT/V)_{actual}$ corresponds to the product of clearance K and treatment time T of the dialysis treatment divided by the distribution volume V of the patient,

determining the clearance $K_{actual}$ and the treatment time $T_{actual}$ of the dialysis treatment, or the product of clearance $K_{actual}$ and treatment time $T_{actual}$,

predetermining a set value of the dialysis dose $(KT/V)_{new}$ for a subsequent dialysis treatment and a determined treatment time $T_{new}$ or a determined clearance $K_{new}$ for the subsequent dialysis treatment,

calculating the clearance $K_{new}$ from the actual value of the dialysis dose $(KT/V)_{actual}$ of the preceding dialysis treatment and the set value of the dialysis dose $(KT/V)_{new}$ for the subsequent dialysis treatment and the treatment time $T_{new}$ of the subsequent dialysis treatment and the clearance $K_{actual}$ and the treatment time $T_{actual}$ of the preceding dialysis treatment, or from the product of clearance $K_{actual}$ and treatment time $T_{actual}$, and predetermining the clearance $K_{new}$ as the new treatment parameter, and/or calculating the treatment time $T_{new}$ from the actual value of the dialysis dose $(KT/V)_{actual}$ of the preceding dialysis treatment and the set value of the dialysis dose $(KT/V)_{new}$ for the subsequent dialysis treatment and the clearance $K_{new}$ of the subsequent dialysis treatment and the clearance $K_{actual}$ and the treatment time $T_{actual}$ of the preceding dialysis treatment, or from the product of clearance $K_{actual}$ and treatment time $T_{actual}$, and predetermining the treatment time $T_{new}$ as the new treatment parameter.

2. The method according to claim 1, **characterized in that** the clearance $K_{new}$ is calculated from the actual value of the dialysis dose $(KT/V)_{actual}$ of the preceding dialysis treatment and the set value of the dialysis dose $(KT/V)_{new}$ for the subsequent dialysis treatment and the treatment time $T_{new}$ of the subsequent dialysis treatment according to the following equation:

$$K_{new} = K_{actual}T_{actual}/T_{new}[(KT/V)_{new}/(KT/V)_{actual}]$$

3. The method according to claim 1 or claim 2, **characterized in that** the clearance $K_{actual}$ of the preceding dialysis treatment is determined from the performance parameters of the dialyser.

4. The method according to claim 1 or claim 2, **characterized in that** the clearance $K_{actual}$ of the preceding dialysis treatment is measured during the dialysis treatment.

5. The method according to any one of the claims 1 to 4, **characterized in that** the actual value of the dialysis dose $(KT/V)_{actual}$ is calculated according to the following equation:

$$K*T/V = -\ln(BUN_{post}/BUN_{pre}-0.008*T) + (4-3.5*BUN_{post}/BUN_{pre})*(UF/W),$$

wherein:

$BUN_{pre}$: predialytic urea concentration in mmol/l
$BUN_{post}$: postdialytic urea concentration in mmol/l
T: dialysis time in h
UF: ultrafiltration volume in l
W: postdialytic weight in kg.

6. A method for setting treatment parameters prior to carrying out an extra-corporal dialysis treatment with a dialyser that is subdivided by a semipermeable membrane into a blood chamber and a dialysis liquid chamber, wherein blood flows at a predetermined blood flow rate $Q_b$ through the blood chamber, and dialysis liquid flows at a predetermined dialysis flow rate $Q_d$ through the dialysis liquid chamber, **characterized in that** the clearance $K_{new}$ is set as new treatment parameter according to the method according to any one of the claims 1 to 5.

7. A device for predetermining treatment parameters for an extra-corporal dialysis treatment with an extra-corporal blood circulation including a dialyser (1) subdivided by a semipermeable membrane (2) into a blood chamber (3) and a dialysis liquid chamber (4), wherein blood flows at a predetermined blood flow rate $Q_b$ through the blood chamber, and dialysis liquid flows at a predetermined dialysis flow rate $Q_d$ through the dialysis liquid chamber, **characterized in that** the device comprises:

an input unit (22) for inputting an actual value of the dialysis dose $(KT/V)_{actual}$ of a preceding dialysis treatment and a set value of the dialysis dose $(KT/V)_{new}$ for a subsequent dialysis treatment and a determined treatment time $T_{new}$, or a determined clearance $K_{new}$ for the subsequent dialysis treatment, and

a calculating unit that is designed such that the clearance $K_{new}$ is calculated from the actual value of the dialysis dose $(KT/V)_{actual}$ of the preceding dialysis treatment and the set value of the dialysis dose $(KT/V)_{new}$ for the subsequent dialysis treatment and the treatment time $T_{new}$ of the subsequent dialysis treatment and the clearance $K_{actual}$ and the treatment time $T_{actual}$ of the preceding dialysis treatment, or from the product of clearance $K_{actual}$ and treatment time $T_{actual}$, and/or is designed such that the treatment time $T_{new}$ is calculated from the actual value of the dialysis dose $(KT/V)_{actual}$ of the preceding dialysis treatment and the set value of the dialysis dose $(KT/V)_{new}$ for the subsequent dialysis treatment and the clearance $K_{new}$ of the subsequent dialysis treatment and the clearance $K_{actual}$ and the treatment time $T_{actual}$ of the preceding dialysis treatment, or from the product of clearance $K_{actual}$ and treatment time $T_{actual}$.

8. The device according to claim 7, **characterized in that** the calculating unit (20) is designed such that the clearance $K_{new}$ is calculated from the actual value of the dialysis dose $(KT/V)_{actual}$ of the preceding dialysis treatment and the set value of the dialysis dose $(KT/V)_{new}$ for the subsequent dialysis treatment and the treatment time $T_{new}$ for the subsequent dialysis treatment according to the following equation:

$$K_{new} = K_{actual} T_{actual} / T_{new} [(KT/V)_{new} / (KT/V)_{actual}]$$

9. The device according to claim 7 or claim 8, **characterized in that** the device further comprises a storage unit (16') for storing the respective clearance $K_{actual}$ of the dialysis treatment for a number of dialysers with different performance parameters.

10. The device according to claim 7 or claim 8, **characterized in that** the device further comprises a measuring and evaluating unit (16) for measuring the clearance $K_{actual}$ of the preceding dialysis treatment.

11. A device for extra-corporal dialysis treatment with a dialyser (1) that is subdivided by a semipermeable membrane (2) into a blood chamber (3) and a dialysis liquid chamber (4), wherein blood flows at a predetermined blood flow rate $Q_b$ through the blood chamber, and dialysis liquid flows at a predetermined dialysis flow rate $Q_d$ through the dialysis liquid chamber, **characterized in that** the dialysis device comprises a device according to any one of the claims 7 to 10.

12. The device for extra-corporal dialysis treatment according to claim 11, **characterized in that** the control unit (13) is designed such that after calculating the clearance $K_{new}$ for the subsequent dialysis treatment at a predetermined blood flow rate $Q_b$, the dialysis liquid rate $Q_d$ is calculated, or at a predetermined dialysis liquid rate $Q_d$, the blood flow rate $Q_b$ is calculated solely on the basis of a predetermined dependency of the clearance on the dialysis liquid flow rate and the blood flow rate at which the clearance predetermined for the subsequent dialysis treatment is maintained.

13. A computer program product comprising a multiplicity of instructions for operating a computer in such a manner that the method steps according to any one of the claims 1 to 6 are carried out.

## Revendications

1. Procédé de prédétermination de paramètres de traitement pour un traitement par dialyse extracorporelle avec un dialyseur qui est subdivisé par une membrane semi-perméable en une chambre de sang et une chambre de liquide de dialyse, dans lequel la chambre de sang est traversée par du sang à un débit de sang prédéterminé $Q_b$ et la chambre de liquide de dialyse est traversée par du liquide de dialyse à un taux de liquide de dialyse prédéterminé $Q_d$, comprenant les étapes du procédé suivantes :

détermination de la valeur instantanée de la dose de dialyse d'un traitement par dialyse, la dose de dialyse $(KT/V)_{instantanée}$ correspondant au produit de la clairance $K$ et du temps de traitement $T$ du traitement par dialyse, divisé par le volume de répartition $V$ du patient,
détermination de la clairance $K_{instantanée}$ et du temps de traitement $T_{instantané}$ du traitement par dialyse ou du produit de la clairance $K_{instantanée}$ et du temps de traitement $T_{instantané}$,

prédétermination d'une valeur de consigne de la dose de dialyse $(KT/V)_{nouv}$ pour un traitement par dialyse consécutif et d'un temps de traitement déterminé $T_{nouv}$ ou d'une clairance déterminée $K_{nouv}$ pour le traitement par dialyse consécutif,

calcul de la clairance $K_{nouv}$ à partir de la valeur instantanée de la dose de dialyse $(KT/V)_{instantanée}$ du traitement par dialyse précédent et de la valeur de consigne de la dose de dialyse $(KT/V)_{nouv}$ pour le traitement par dialyse consécutif et du temps de traitement $T_{nouv}$ du traitement par dialyse consécutif et de la clairance $K_{instantanée}$ et du temps de traitement $T_{instantané}$ du traitement par dialyse précédent ou du produit de la clairance $K_{instantanée}$ et du temps de traitement $T_{instantané}$, et

prédétermination de la clairance $K_{nouv}$ en tant que nouveau paramètre de traitement et/ou

calcul du temps de traitement $T_{nouv}$ à partir de la valeur instantanée de la dose de dialyse $(KT/V)_{instantanée}$ du traitement par dialyse précédent et de la valeur de consigne de la dose de dialyse $(KT/V)_{nouv}$ pour le traitement par dialyse consécutif et de la clairance $K_{nouv}$ du traitement par dialyse consécutif et de la clairance $K_{instantanée}$ et du temps de traitement $T_{instantané}$ du traitement par dialyse précédent ou du produit de la clairance $K_{instantanée}$ et du temps de traitement $T_{instantané}$, et

prédétermination du temps de traitement $T_{nouv}$ en tant que nouveau paramètre de traitement.

**2.** Procédé selon la revendication 1, **caractérisé en ce que** la clairance $K_{nouv}$ est calculée à partir de la valeur instantanée de la dose de dialyse $(KT/V)_{instantanée}$ du traitement par dialyse précédent et de la valeur instantanée de la dose de dialyse $(KT/V)_{nouv}$ pour le traitement par dialyse consécutif et du temps de traitement $T_{nouv}$ du traitement par dialyse consécutif selon l'équation suivante :

$$K_{nouv} = K_{instantanée} T_{instantané}/T_{nouv}[(KT/V)_{nouv}/(KT/V)_{instantanée}]$$

**3.** Procédé selon la revendication 1 ou 2, **caractérisé en ce que** la clairance $K_{instantanée}$ du traitement par dialyse précédent est réalisée à partir des paramètres de performance du dialyseur.

**4.** Procédé selon la revendication 1 ou 2, **caractérisé en ce que** la clairance $K_{instantanée}$ du traitement par dialyse précédent est mesurée pendant le traitement par dialyse.

**5.** Procédé selon l'une des revendications 1 à 4, **caractérisé en ce que** la valeur instantanée de la dose de dialyse $(KT/V)_{instantanée}$ du traitement par dialyse précédent est calculée selon l'équation suivante

$$K*T/V = -\ln (BUN_{post}/BUN_{pré} -0{,}008*T) + (4-3{,}5*BUN_{post}/BUN_{pré})*(UF/W),$$

où

BUN pré : concentration d'urée pré-dialytique en mmol/l
BUN post : concentration d'urée post-dialytique en mmol/l
T : temps de dialyse en h
UF : volume d'ultrafiltration en l
W : poids post-dialytique en kg.

**6.** Procédé d'ajustement de paramètres de traitement avant la réalisation d'un traitement par dialyse extracorporelle avec un dialyseur qui est subdivisé par une membrane semi-perméable en une chambre de sang et une chambre de liquide de dialyse, dans lequel la chambre de sang est traversée par du sang à un débit de sang prédéterminé $Q_b$ et la chambre de liquide de dialyse est traversée par du liquide de dialyse à un taux de liquide de dialyse prédéterminé $Q_d$, **caractérisé en ce que** la clairance $K_{nouv}$ est ajustée en tant que nouveau paramètre de traitement selon le procédé selon l'une des revendications 1 à 5.

**7.** Dispositif de prédétermination de paramètres de traitement pour un traitement par dialyse extracorporelle avec une circulation sanguine extracorporelle qui contient un dialyseur (1) qui est subdivisé par une membrane semi-perméable (2) en une chambre de sang (3) et une chambre de liquide de dialyse (4), dans lequel la chambre de sang est traversée par du sang à un débit de sang prédéterminé $Q_b$ et la chambre de liquide de dialyse est traversée par du liquide de dialyse à un taux de liquide de dialyse prédéterminé $Q_d$,

**caractérisé en ce que** le dispositif présente :

une unité d'entrée (22) pour entrer une valeur instantanée de la dose de dialyse $(KT/V)_{instantanée}$ d'un traitement par dialyse précédent et une valeur de consigne de la dose de dialyse $(KT/V)_{nouv}$ pour un traitement par dialyse consécutif et d'un temps de traitement $T_{nouv}$ déterminé ou d'une clairance déterminée $K_{nouv}$ pour le traitement par dialyse consécutif et

une unité de calcul qui est conçue de telle sorte que la clairance $K_{nouv}$ est calculée à partir de la valeur instantanée de la dose de dialyse $(KT/V)_{instantanée}$ du traitement par dialyse précédent et de la valeur de consigne de la dose de dialyse $(KT/V)_{nouv}$ pour le traitement par dialyse consécutif et de la clairance $K_{instantanée}$ et du temps de traitement $T_{instantané}$ du traitement par dialyse précédent ou du produit de la clairance $K_{instantanée}$ et du temps de traitement $T_{instantané}$

et/ou est conçue de telle sorte que le temps de traitement $T_{nouv}$ est calculé à partir de la valeur instantanée de la dose de dialyse $(KT/V)_{instantanée}$ du traitement par dialyse précédent et de la valeur de consigne de la dose de dialyse $(KT/V)_{nouv}$ pour le traitement par dialyse consécutif et de la clairance $K_{nouv}$ du traitement par dialyse consécutif et de la clairance $K_{instantanée}$ et du temps de traitement $T_{instantané}$ du traitement par dialyse précédent ou du produit de la clairance $K_{instantanée}$ et du temps de traitement $T_{instantané}$.

8. Dispositif selon la revendication 7, caractérisé en ce que l'unité de calcul (20) est conçue de telle sorte que la clairance $K_{nouv}$ est calculée à partir de la valeur instantanée de la dose de dialyse $(KT/V)_{instantanée}$ du traitement par dialyse précédent et de la valeur instantanée de la dose de dialyse $(KT/V)_{nouv}$ pour le traitement par dialyse consécutif et du temps de traitement $T_{nouv}$ pour le traitement par dialyse consécutif selon l'équation suivante :

$$K_{nouv} = K_{instantanée} T_{instantané}/T_{nouv} [(KT/V)_{nouv}/(KT/V)_{instantané}]$$

9. Dispositif selon la revendication 7 ou 8, **caractérisé en ce que** le dispositif présente en outre une unité de mémoire (16') pour mémoriser la clairance $K_{instantanée}$ respective du traitement par dialyse pour un nombre de dialyseurs comportant des paramètres de performance différents.

10. Dispositif selon la revendication 7 ou 8, **caractérisé en ce que** le dispositif présente en outre une unité de mesure et d'analyse (16) pour mesurer la clairance $K_{instantanée}$ du traitement par dialyse précédent.

11. Dispositif de traitement par dialyse extra-corporelle comportant un dialyseur (1) qui est subdivisé par une membrane semi-perméable (2) en une chambre de sang (3) et une chambre de liquide de dialyse (4), la chambre de sang étant traversée par du sang à un débit de sang pré-déterminé $Q_b$ et la chambre de liquide de dialyse étant traversée par du liquide de dialyse à un taux de liquide de dialyse prédéterminé $Q_d$, **caractérisé en ce que** le dispositif de dialyse présente un dispositif selon l'une des revendications 7 à 10.

12. Dispositif de traitement par dialyse extra-corporelle selon la revendication 11, caracté-risé en ce que l'unité de commande (13) est conçue de telle sorte qu'après le calcul de la clairance $K_{nouv}$ pour le traitement par dialyse consécutif à un débit de sang $Q_b$ prédéterminé, le taux de liquide de dialyse $Q_d$ ou à un taux de liquide de dialyse prédéterminé $Q_d$, le débit sanguin $Q_b$ seul est calculé sur la base d'une dépendance prédéterminée de la clairance du taux de liquide de dialyse et du débit sanguin à laquelle la clairance prédéterminée pour le traitement par dialyse consécutif est conservée.

13. Logiciel informatique comportant une multi-plicité d'instructions pour faire fonctionner un ordinateur de telle sorte que les étapes du procédé selon l'une des revendications 1 à 6 soient réalisées.

Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0428927 A1
- EP 1062960 A2
- DE 102006026999
- DE 102006038545

**In der Beschreibung aufgeführte Nicht-Patentliteratur**

- **KLOPPENBURG W ; STEGEMANN C et al.** Anthropometrybased equations overestimate the urea distribution volume in hemodialysis patients. *Kidney Int.,* 2001, vol. 59, 1165-1174
- **DAUGIRDAS, J.** Second generation logarithmic estimates of single-pool variable volume kt/v: an analysis of error. *J. Am. Soc. Nephrol.,* 1993, vol. 4, 1205-1213